# EUROPÄISCHE PATENTSCHRIFT

(11) **EP 2 366 721 B1**
(45) Veröffentlichungstag und Bekanntmachung des Hinweises auf die Patenterteilung: **10.08.2016**
(21) Anmeldenummer: 11158496.7
(22) Anmeldetag: 16.03.2011
(51) Int. Cl.: C08F 22/32, A61L 24/06

(54) **Medizinisches Kit zur Anwendung bei der Vakuumversiegelungstherapie**
Medical kit for use during negative pressure wound therapy
Kit médical pour l'application dans le cadre de la thérapie de scellement sous vide

(30) Priorität: 17.03.2010 DE 102010012521
(43) Veröffentlichungstag der Anmeldung: 21.09.2011
(73) Patentinhaber: AESCULAP AG, 78532 Tuttlingen (DE)
(72) Erfinder: Odermatt, Erich, 8200, Schaffhausen (CH); Siedle, Gabriel, 79117, Freiburg (DE); Wegmann, Jürgen, 78333, Stockach (DE)
(74) Vertreter: Patentanwälte Ruff, Wilhelm, Beier, Dauster & Partner mbB

(56) Entgegenhaltungen:
- EP-A1- 1 861 131
- EP-A1- 1 884 223
- WO-A1-03/008003

## Beschreibung

Die vorliegende Erfindung betrifft ein medizinisches Kit, welches sich insbesondere zur Vakuumversiegelung und -drainage eignet.

Unter dem Begriff Wunde versteht man allgemein eine durch äußere Einflüsse entstandene, umschriebene oder flächenhafte Gewebsdurchtrennung oder Zerstörung. Derartigen Wunden liegen im Wesentlichen mechanische, thermische, chemische oder strahlenbedingte Ursachen zugrunde. So können Wunden durch Operationen, Verletzungen oder chronische Vorgänge wie zum Beispiel Diabetes entstehen.

Man unterscheidet allgemein zwischen einfachen, nur die Oberfläche eines Gewebes betreffende Wunden und komplexe Wunden mit Ausdehnung in Muskel-, Knochen- und Nervengewebe. Die adäquate Versorgung von Wunden entscheidet maßgeblich über das mögliche Heilungsergebnis.

In Bezug auf den Wundheilungsverlauf wird weiterhin zwischen einer primären und einer sekundären Wundheilung unterschieden. Eine primäre Wundheilung (sanatio per primam intentionem) ist dann möglich, wenn die Wundränder lückenlos adaptiert sind, wie beispielsweise bei der klassischen chirurgischen Wunde, und der Gewebsdefekt durch minimales, neu gebildetes Bindegewebe, sogenanntes Granulationsgewebe, ersetzt wird.

Eine sekundäre Wundheilung (sanatio per secundam intentionem) tritt dann ein, wenn sich die Wundränder nicht adaptieren lassen oder eine medizinische Indikation gegen die Adaptation spricht (infizierte Wunde oder Ähnliches). In diesem Fall wird der Wunddefekt langsam durch Granulationsgewebe aufgefüllt, und es entsteht eine Narbe. Beispielsweise unterliegt jede chronische Wunde einer sekundären Wundheilung.

Zur Wundversorgung existieren mittlerweile zahlreiche Behandlungsmethoden. Die Auswahl reicht von Binden, Schlauch- und Netzschlauchverbänden über Kompressionsverbände bis zu Stützverbänden und ruhigstellenden Verbänden.

Ein vielversprechender Ansatz zur Wundtherapie stellt die sogenannte Vakuumversiegelung (V.A.C. Vacuum Assisted Closure) dar. Das Prinzip der Vakuumversiegelung besteht darin, durch einen offenporigen Schwamm die Vakuumwirkung einer Saugdrainage großflächig auf die gesamte Wundoberfläche zu verteilen. Hierdurch wird einerseits die Wundflüssigkeit (Exsudat) einschließlich der darin enthaltenen Bakterien und toxischen Zerfallsprodukte kontinuierlich aus dem Wundmilieu abgeführt, wodurch eine effektive Wundreinigung zustande kommt. Andererseits regt das Vakuum bzw. der Unterdruck Wundheilungsprozesse an und führt insbesondere zu einer gesteigerten und schnelleren Bildung von gesundem Granulationsgewebe, einer verbesserten mikrovaskulären Blutflussdynamik sowie zu einer Reduzierung von interstitiellen Wundödemen.

Ein grundsätzliches Problem der Vakuumversiegelung besteht jedoch darin, dass häufig eine nur unzureichende, in manchen Fällen sogar gar keine Kontrolle über das angelegte Vakuum bzw. den angelegten Unterdruck möglich ist. Hieraus können sich unerwünschte Folgekomplikationen ergeben. Wird beispielsweise ein zu starker Unterdruck angelegt, kann dies schmerzhaft für den Patienten, insbesondere beim Verbandwechsel, sein. Wird der Unterdruck dagegen zu niedrig festgelegt, kann es zu einem Wundflüssigkeitsstau zwischen Wundoberfläche und Drainageschwamm kommen, wodurch das Risiko von sekundären Infektionen und unerwünschten Gewebereaktionen, wie Gewebemazerationen, steigt.

Cyanoacrylat-basierte Kleber für medizinische Anwendungen, insbesondere zur Verklebung bzw. Versiegelung von Wunden, sind aus der WO 2006/102385 A1 sowie WO 03/008003 A1 bekannt.

Gegenstand der EP 1 884 223 A1 ist eine wundstimulierende Vorrichtung, welche im Rahmen der Vakuumversiegelung eingesetzt werden und einen hydrophilen, synthetischen Schwamm aufweisen kann.

Die Erfindung stellt sich daher die Aufgabe, ein medizinisches Kit für die Wunddrainage im Rahmen einer Vakuumversiegelung bereitzustellen, welche aus dem Stand der Technik bekannte Unzulänglichkeiten vermeidet und insbesondere eine im Verhältnis zum Stand der Technik zuverlässigere Kontrolle des angelegten Vakuums bzw. Unterdrucks ermöglicht.

Diese Aufgabe wird erfindungsgemäß gelöst durch ein medizinisches Kit, insbesondere zur Vakuumversiegelung und Vakuumdrainage von Wunden, umfassend die folgenden Komponenten:
- 1.1: einen Saugkörper zum Aufsaugen von Körperflüssigkeiten, insbesondere Blut, Wundflüssigkeiten oder dergleichen,
- 1.2: eine polymerisierbare Klebstoffzusammensetzung und
- 1.3: einen Polymerisationsinitiator bzw. Aushärter für die Klebstoffzusammensetzung.

Unter einem Saugkörper soll im Sinne der vorliegenden Erfindung ein Formkörper verstanden werden, welcher in der Lage ist, Körperflüssigkeiten, insbesondere Blut, Wundflüssigkeiten (Wundsekrete bzw. Exsudate) oder dergleichen, aufzusaugen. In der Regel wird das Aufsaugen von Körperflüssigkeiten durch Kapillarkräfte des Saugkörpers ermöglicht.

Unter einem Polymerisationsinitiator soll im Sinne der vorliegenden Erfindung eine Verbindung verstanden werden, welche die Polymerisation von in der Klebstoffzusammensetzung enthaltenen, polymerisierbaren Komponenten, besonders bevorzugt von Cyanoacrylat-Monomeren zu einem Polycyanoacrylat, initiiert und den Polymerisationsvorgang gegebenenfalls auch beschleunigt und/oder katalysiert. Demzufolge kann es sich bei dem Polymerisationsinitiator im Sinne der vorliegenden Erfindung auch um einen Polymerisationsbeschleuniger und/oder Katalysator handeln.

Überraschenderweise konnte festgestellt werden, dass sich eine Kombination auf Basis eines Saugkörpers, einer polymerisierbaren Klebstoffzusammensetzung sowie vorzugsweise eines Polymerisationsinitiators hervorragend dazu eignet, eine Wunde im Wesentlichen luftdicht zu verschließen, weswegen sich die Kombination insbesondere zur Wundtherapie mittels Vakuumversiegelung eignet. Durch eine vorzugsweise vollflächige Polymerisation bzw. Aushärtung der Klebstoffzusammensetzung auf dem Saugkörper bildet sich ein im Wesentlichen luftundurchlässiger, filmartiger Überzug auf dem Saugkörper sowie an dessen Randzonen aus. Dadurch wird ein luftdichter Verschluss einer unter dem Saugkörper liegenden Wunde sowie eine Fixierung des Saugkörpers an den Wundrändern erzielt. Hierdurch lassen sich an den Saugkörper im Hinblick auf einen günstigen Wundheilungsverlauf optimale und insbesondere kontrollierbare Unterdrücke anlegen, wodurch die Vorteile der Vakuumversiegelung, insbesondere im Hinblick auf die Entstehung von Granulationsgewebe, Verbesserung der mikrovaskulären Blutflussdynamik im Wundareal sowie die anti-ödematöse Wirkung, besser zur Entfaltung kommen können.

In einer bevorzugten Ausführungsform ist der Saugkörper mindestens teilweise, vorzugsweise vollständig, porös, in der Regel offenporös, ausgebildet. Mit anderen Worten ist es bevorzugt, wenn der Saugkörper eine poröse, insbesondere offenporöse, Struktur besitzt.

Der Saugkörper kann grundsätzlich eine Porengröße zwischen 50 und 1.500 pm besitzen. Bevorzugt ist eine Porengröße zwischen 150 und 1.200 µm, insbesondere 200 und 1.000 µm, vorzugsweise 250 und 650 µm.

Der Saugkörper weist vorzugsweise ein Saugvermögen für Körperflüssigkeiten auf, welches dem 10- bis 100-fachen, insbesondere 20- bis 40-fachen, seines trockenen Eigengewichtes entspricht. Zur Erhöhung der Saugfähigkeit des Saugkörpers kann dieser saugfähigkeitsverbessernde Additive, insbesondere Superabsorbenzien, beispielsweise vernetzte Polyacrylsäuresalze bzw. vernetzte Polyelektrolyte, aufweisen.

Der Saugkörper ist bei einer weiteren Ausführungsform elastisch bzw. reversibel komprimierbar ausgebildet. Besonders vorteilhaft ist es, wenn sich der Saugkörper bei Kontakt mit Körperflüssigkeiten, insbesondere Wundflüssigkeiten, ausdehnt, um die gesamte Fläche einer Wunde abzudecken.

Der Saugkörper ist in einer weiteren Ausführungsform als Folie, Schwamm oder Schaum ausgebildet. Erfindungsgemäß ist es besonders bevorzugt, wenn der Saugkörper ein Schwamm oder Schaum ist.

In einer weitergehenden Ausführungsform ist eine Seite des Saugkörpers mit einer luftundurchlässigen Schicht bzw. Beschichtung versehen. Eine zu der einen Seite gegenüberliegende Seite des Saugkörpers ist vorzugsweise frei von einer solchen Schicht bzw. Beschichtung. Die Beschichtung kann beispielsweise film- oder folienartig ausgebildet sein. Mit anderen Worten kann es erfindungsgemäß vorgesehen sein, dass der Saugkörper auf einer Seite mit einem Film oder einer Folie versehen, insbesondere bedeckt bzw. überzogen, ist. Die unbeschichtete Seite des Saugkörpers ist in der Regel zur unmittelbaren Auflage auf eine Wunde vorgesehen. Ein einseitig luftundurchlässig beschichteter Saugkörper hat den Vorteil, dass insgesamt eine geringere Menge an Klebstoffzusammensetzung erforderlich ist, um den Saugkörper nach Auflage auf eine Wunde luftdicht zu versiegeln.

Grundsätzlich kann der Saugkörper aus jedem beliebigen, bioverträglichen Material gebildet sein bzw. auf einem derartigen Material basieren. Unter einem bioverträglichen Material im Sinne der vorliegenden Erfindung soll dabei ein körperverträgliches, insbesondere gewebeverträgliches, Material verstanden werden. Das Material kann resorbierbar, teilresorbierbar oder nicht resorbierbar sein. Bevorzugt ist der Saugkörper aus einem polymeren Material, insbesondere einem Biopolymer, d.h. einem natürlich vorkommenden Polymer, einem davon abgeleiteten Polymer oder einem synthetischen Polymer, insbesondere Homo- oder Copolymer, gebildet. Als Copolymere kommen grundsätzlich statistische Copolymere, Gradientcopolymere, alternierende Copolymere, Blockcopolymere und/oder Pfropfcopolymere in Betracht. Unter einem Copolymer im Sinne der vorliegenden Erfindung soll dabei ein Polymer verstanden werden, welches aus zwei oder mehr verschiedenartigen Monomereinheiten zusammengesetzt ist.

In einer weitergehenden Ausführungsform basiert der Saugkörper auf einem bioverträglichen Polymer, welches ausgewählt ist aus der Gruppe bestehend aus Polyurethane, Polysiloxane, Polyvinylalkohole, Polyester, insbesondere Polyethylenterephthalat und/oder Polybutylenterephthalat, Polyhydroxyalkanoate, Polysaccharide, insbesondere Cellulose, Cellulosederivate, zum Beispiel Alkylcellulosen, Mucopolysaccharide, Salze davon, Copolymere davon und Mischungen, insbesondere Blends, davon.

Geeignete Polysaccharide können zum Beispiel aus der Gruppe bestehend aus Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Alginsäure, Stärke, Amylose, Amylopektin, Dextran, Chitosan, Hyaluronsäure, Heparin, Heparansulfat, Chondroitin-4-sulfat, Chondroitin-6-sulfat, Dermatansulfat, Keratansulfat, Salze davon, Derivate davon und Kombinationen davon ausgewählt sein.

Besonders bevorzugt ist es, wenn der Saugkörper ein Polyurethan, insbesondere ein elastisches oder thermoplastisches Polyurethan, aufweist. Bevorzugt weist der Saugkörper ein lineares und/oder aliphatisches Polyurethan, besonders bevorzugt ein lineares und aliphatisches Polyurethan, auf. Das Polyurethan ist vorzugsweise ausgewählt aus der Gruppe bestehend aus Polycarbonat-Polyurethane, Polyester-Polyurethane, Polysiloxan-Polyurethane, Polycarbonat-Polyester-Polyurethane, Polycarbonat-Polyether-Polyurethane, Polycarbonat-Polysiloxan-Polyurethane, Polyester-Polyether-Polyurethane, Polyester-Polysiloxan-Polyurethane, Polyether-Polysiloxan-Polyurethane, Copolymere davon und Mischungen davon.

Polyurethan ist wegen seiner ausgezeichneten Bioverträglichkeit in besonderem Maße als Saugkörpermaterial geeignet. Das Polyurethan kann insbesondere aus makromolekularen und/oder niedermolekularen aliphatischen Diolen und vorzugsweise aliphatischen Diisocyanaten hergestellt sein. Geeignete makromolekulare Diole sind vor allem Polycarbonate, insbesondere 1,6-Hexandiolpolycarbonat. Bevorzugte niedermolekulare Diole, von denen das Polyurethan abgeleitet sein kann, sind beispielsweise 2,2,4-Trimethylhexandiol, 2,4,4-Trimethylhexandiol und/oder 1,4-Butandiol. Als aliphatische Diisocyanate zur Herstellung des Polyurethans können acyclische aliphatische und/oder cycloaliphatische Diisocyanate, insbesondere 4,4-Dicyclohexylmethandiisocyanat und/oder 1,4-Cyclohexyldiisocyanat, verwendet werden.

In einer alternativen, ebenfalls bevorzugten Ausführungsform basiert der Saugkörper auf einem Polyurethanderivat, insbesondere einem Polyurethanether, oder einem Polyurethancopolymer, beispielsweise einem Silicon-Polyurethan-Copolymer.

In einer weiteren Ausführungsform weist der Saugkörper ein resorbierbares Polymer, beispielsweise ein Polyhydroxyalkanoat, auf. Bevorzugt weist der Saugkörper ein resorbierbares Polymer auf, welches aus der Gruppe bestehend aus Polylactid, Polyglycolid, Poly-ε-caprolacton, Trimethylencarbonat, Poly-para-dioxanon, Poly-3-hydroxybutyrat, Poly-4-hydroxybutyrat, Copolymere davon und Mischungen davon ausgewählt ist.

Erfindungsgemäß kann es weiterhin vorgesehen sein, dass der Saugkörper eine Kombination oder Mischung der in den vorhergehenden Ausführungsformen beschriebenen Materialien aufweist. Insbesondere kann der Saugkörper aus einem der in den vorhergehenden Ausführungsformen beschriebenen Materialien oder aus einer Kombination bzw. Mischung dieser Materialien bestehen bzw. hergestellt sein.

In der Regel weist der Saugkörper einen Drainageschlauch zur Abführung bzw. Ableitung (Drainage) der vom Saugkörper aufgesogenen Körperflüssigkeiten auf. Auf diese Weise ist zum Einen eine Saugdrainage der zu behandelnden Wunde möglich. Zum Anderen lässt sich hierdurch das ursprüngliche Saugvermögen des Saugkörpers wiederhergestellen. Der Drainageschlauch ist gewöhnlich aus einem Kunststoffmaterial, beispielsweise Polyethylen, Polypropylen, Polyvinylchlorid oder Polyurethan, gebildet.

Der Saugkörper ist in einer weitergehenden Ausführungsform einstückig mit einem Drainageschlauch verbunden. Bevorzugt ist es, wenn ein Ende des Drainageschlauches von dem Saugkörper umgeben bzw. ummantelt ist. Hierzu kann der Saugkörper an den Drainageschlauch angeformt sein. Beispielsweise kann der Saugkörper mit dem Drainageschlauch verklebt, vernäht, verschweißt oder auf den Drainageschlauch aufgeschäumt sein. Mit anderen Worten ist es bevorzugt, wenn ein Teil des Drainageschlauches in den Saugkörper, bevorzugt in eine offenporöse Struktur des Saugkörpers, hineinragt.

Besonders bevorzugt ist es, wenn der Saugkörper eine sich in dessen Inneres erstreckende schlauchförmige Ausnehmung zur Aufnahme eines Drainageschlauchendes aufweist. Die Ausnehmung erstreckt sich vorzugsweise in etwa mittig des Saugkörpers und vorzugsweise in dessen Längsrichtung. Die Ausnehmung besitzt zweckmäßigerweise einen Durchmesser, der dem Durchmesser des Drainageschlauches angepasst ist.

Vorzugsweise wird ein perforiertes Ende des Drainageschlauches von dem Saugkörper umgeben bzw. ummantelt. Auf diese Weise ist ein Flüssigkeitstransfer vom Saugkörper in den Drainageschlauch und damit eine Ableitung von Flüssigkeiten aus einem Wundmilieu möglich.

Um eine Vakuumversiegelung durchführen zu können, ist der Drainageschlauch über sein anderes Ende in der Regel an eine Saug- oder Vakuumpumpe anschließbar. Gewöhnlich kommen hierbei elektronische Saug- oder Vakuumpumpen oder Vakuumflaschen ("Redonflaschen") zum Einsatz. Bei den Saug- bzw. Vakuumpumpen kann es sich insbesondere um tragbare Vakuumpumpen handeln. Auf diese Weise kann die Mobilität eines Patienten während einer Vakuumversiegelungstherapie aufrechterhalten werden. Die Vakuumversiegelungstherapie kann mehrere Stunden, gegebenenfalls auch mehrere Tage, dauern. Die Ableitung bzw. Drainage von Körperflüssigkeiten aus einer Wunde kann grundsätzlich intermittierend, insbesondere zeitintervallgesteuert, erfolgen. Bevorzugt erfolgt eine kontinuierliche Saugdrainage, um zu verhindern, dass sich Wundflüssigkeit bzw. Exsudat im Wundmilieu ansammelt. Die entfernten Flüssigkeitsmengen werden üblicherweise in hierfür vorgesehenen Sammelbehältern, beispielsweise Kanistern oder Vakuumflaschen, aufgefangen. Die Sammelbehälter sind in der Regel den Saug- oder Vakuumpumpen vorgeschaltet und stehen mit diesen über geeignete Verbindungsschläuche in Kontakt. Um die Saug- bzw. Vakuumpumpen nicht zu kontaminieren, können zwischen den Sammelbehältern und den Saug- bzw. Vakuumpumpen sterile Filter vorgesehen sein.

Der Saugkörper weist in einer weiteren Ausführungsform einen Spülschlauch auf. Der Spülschlauch dient in aller Regel einem gezielten Anspülen des Saugkörpers, wodurch dieser in vorteilhafter Weise flexibler und insbesondere geschmeidiger gemacht werden kann. Als Spülflüssigkeiten können körperverträgliche Salzlösungen, Pufferlösungen, Elektrolytlösungen und gegebenenfalls auch entzündungshemmende, geruchshemmende und/oder antimikrobiell wirksame Lösungen verwendet werden. Grundsätzlich ist die in diesem Absatz beschriebene Spülung des Saugkörpers auch mittels des zuvor beschriebenen Drainageschlauches möglich. Bezüglich weiterer Merkmale Vorteile des Spülschlauches wird auf die zuvor in Bezug auf den Drainageschlauch beschriebenen Ausführungsformen Bezug genommen.

Der Saugkörper kann grundsätzlich in unterschiedlichen Formen und Größen vorliegen, abhängig von der jeweils zu versorgenden Wunde. Beispielsweise kann der Saugkörper eine kreisförmige, ovale, dreieckige, quadratische, rechteckige, trapezoidale, rhomboide, pentagonale bzw. fünfeckige, sechseckige oder andere Formen, insbesondere polygonale Formen, besitzen. Erfindungsgemäß kann es prinzipiell auch vorgesehen sein, dass der Saugkörper als Hohlkörper, beispielsweise als Schlauch, Rohr oder Hohlzylinder, ausgebildet ist.

Die Klebstoffzusammensetzung weist als polymerisierbare Komponente ein Cyanoacrylat-Monomer gemäß untenstehender Formel (I) oder eine Mischung eines Cyanoacrylat-Monomers gemäß Formel (I) (allgemeine Strukturformel eines Cyanoacrylat-Monomers) mit weiteren Cyanoacrylat-Monomeren auf, wobei R eine substituierte oder unsubstituierte, geradkettige, verzweigte oder zyklische Alkyl-, Haloalkyl-, Alkoxyalkyl-, Alkenyl-, Allyl- oder Alkinyl-Gruppe ist, welche bevorzugt 1 bis 18 Kohlenstoffatome, weiter bevorzugt 2 bis 12 Kohlenstoffatome, besonders bevorzugt 4 bis 8 Kohlenstoffatome, umfasst, und/oder eine aromatische Gruppe und/oder eine Acylgruppe aufweist.

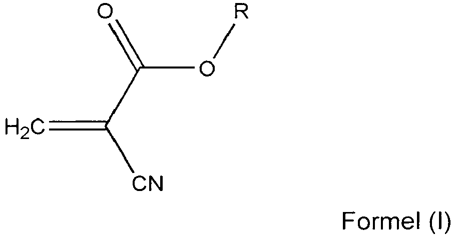

Erfindungsgemäß bevorzugt sind Alkylcyanoacrylat-Monomere, Alkoxyalkylcyanoacrylat-Monomere, Alkylestercyanoacrylat-Monomere und Mischungen davon.

In einer weitergehenden Ausführungsform weist die Klebstoffzusammensetzung als polymerisierbare Komponente ein Cyanoacrylat-Monomer auf, welches aus der Gruppe bestehend aus Allyl-2-cyanoacrylat, β-Methoxy-ethyl-2-cyanoacrylat, Methyl-2-cyanoacrylat, Ethyl-2-cyanoacrylat, n-Propyl-2-cyanoacrylat, Isopropyl-2-cyanoacrylat, n-Butyl-2-cyanoacrylat, Isobutyl-2-cyanoacrylat, n-Pentyl-2-cyanoacrylat, Isopentyl-2-cyanoacrylat, Cyclopentyl-2-cyanoacrylat, n-Hexyl-2-cyanoacrylat, Iso-hexyl-2-cyanoacrylat, Cyclohexyl-2-cyanoacrylat, n-Heptyl-2-cyanoacrylat, Isoheptyl-2-cyanoacrylat, Cycloheptyl-2-cyanoacrylat, n-Octyl-2-cyanoacrylat, Dodecyl-2-cyanoacrylat, Lactoyl-2-cyanoacrylat, Methoxyisopropyl-2-cyanoacrylat, Ethoxyethyl-2-cyanoacrylat, Isopropoxyethyl-2-cyanoacrylat, 2-Butoxyethyl-2-cyanoacrylat, Butyllactoyl-2-cyanoacrylat und Kombinationen davon ausgewählt ist. Besonders bevorzugte Ausführungsformen umfassen n-Butyl-2-cyanoacrylat, n-Octyl-2-cyanoacrylat, Ethoxyethyl-2-cyanoacrylat oder Kombinationen davon.

In einer besonders bevorzugten Ausführungsform weist die Klebstoffzusammensetzung ferner einen Weichmacher auf. Durch den Weichmacher erhält die polymerisierte bzw. ausgehärtete Klebstoffzusammensetzung eine größere Flexibilität und Weichheit. Dadurch ist es der ausgehärteten bzw. polymerisierten Klebstoffzusammensetzung möglich, kontrahierende und expandierende Bewegungen des Saugkörpers während der Vakuumversiegelungstherapie mitzumachen, ohne dass die polymerisierte Klebstoffzusammensetzung von dem Saugkörper abbröckelt bzw. abblättert, was den luftdichten Verschluss des Saugkörpers während der Vakuumversiegelung gefährden würde.

Grundsätzlich kann die Klebstoffzusammensetzung einen Weichmacheranteil von bis zu 80 Gew.-%, bezogen auf das Gesamtgewicht der Klebstoffzusammensetzung, aufweisen. Bevorzugt ist es, wenn die Klebstoffzusammensetzung einen Weichmacheranteil zwischen 5 und 75 Gew.-%, insbesondere 10 und 70 Gew.-%, vorzugsweise 15 und 50 Gew.-%, bezogen auf das Gesamtgewicht der Klebstoffzusammensetzung, aufweist.

Bei dem Weichmacher handelt es sich bevorzugt um Esterverbindungen, insbesondere organische Esterverbindungen, Etherverbindungen oder um Kombinationen davon.

Bevorzugte Esterverbindungen sind aus der Gruppe bestehend aus Ethylacetat, Butylacetat, Glycerintriacetat, Glycerintripropionat, Triglycerinpentaacetat, Polyglycerinacetat, Diethylenglycoldiacetat, 3-Hydroxyvaleriansäureethylester, Milchsäurebutylester, Milchsäureisobutylester, 3-Hydroxybuttersäureethylester, Oxalsäurediethylester, Mesoxalsäurediethylester, Apfelsäuredimethylester, Apfelsäurediisopropylester, Weinsäurediethylester, Weinsäuredipropylester, Weinsäurediisopropylester, Glutarsäuredimethylester, Bernsteinsäuredimethylester, Bernsteinsäurediethylester, Maleinsäurediethylester, Fumarsäurediethylester, Malonsäurediethylester, Acrylsäure-2-hydroxyethylester, 3-Oxovaleriansäuremethylester, Glycerindiacetat, Glycerintributyrat, Glycerintripropionat, Glycerindipropionat, Glycerintriisobutyrat, Glycerindiisobutyrat, Glycidylbutyrat, Acetessigsäurebutylester, Lävulinsäureethylester, 3-Hydroxyglutarsäuredimethylester, Glycerinacetatdipropionat, Glycerindiacetatbutyrat, Propionsäurebutylester, Propylenglycoldiacetat, Propylenglycoldibutyrat, Diethylenglycoldibutyrat, Trimethylolethantriacetat, Trimethylolpropantriacetat, Trimethylolethantributyrat, Neopentylalkoholdibutyrat, Methoxyessigsäurepentylester, Dimethoxyessigsäurebutylester, Glycolsäurebutylester und Kombinationen davon ausgewählt.

Weitere bevorzugte Weichmacher sind Citronensäureester bzw. Citratester, vorzugsweise mit Alkoholresten mit 1 bis 10 Kohlenstoffatomen. Beispielsweise können die Citronensäure- bzw. Citratester aus der Gruppe bestehend aus Triethylacetylcitrat, Tributylacetylcitrat, Trihexylacetylcitrat und Kombinationen davon ausgewählt sein.

Weitere geeignete Weichmacher sind beispielsweise Ester, welche aus der Gruppe bestehend aus Abietinsäureester, Adipinsäureester, Azelainsäureester, Benzoesäureester, Buttersäureester, Essigsäureester, Fettsäureester, beispielsweise Butylmyristat, Isobutylmyristat, Ethylmyristat, Ethylstearat, Methylsebacat, Ethylsebacat oder Kombinationen davon, Fette, verzweigte Alkohole, Propionsäureester, Sebacinsäureester, Sulfonsäureester, Thiobuttersäureester, Trimellithsäureester, Zitronensäureester, beispielsweise Methylcitrat, Ethylcitrat oder Kombinationen davon, Celluloseester, beispielsweise Ethylcellulose und Kombinationen davon ausgewählt sind.

Bevorzugte Etherverbindungen als Weichmacher stellen reine oder gemischte Ether monofunktioneller, linearer oder verzweigter Alkohole, welche vorzugsweise 4 bis 16 Kohlenstoffatome umfassen, oder Gemische aus 2 oder mehr verschiedenen Ethern derartiger Alkohole, beispielsweise Dioctylether, dar. Des Weiteren eignen sich als Weichmacher auch endgruppenverschlossene Polyethylenglykole. Beispielhaft seien an dieser Stelle Polyethylen- oder Polypropylenglykoldialkylether, insbesondere Polyethylenglykoldimethylether und/oder Polyethylenglykoldiethylether, genannt.

Erfindungsgemäß kann es weiterhin vorgesehen sein, dass der Weichmacher auch eine Kombination aus den in den vorhergehenden Ausführungsformen genannten Weichmachern darstellt.

Um einer vorzeitigen Polymerisation bzw. Aushärtung der Klebstoffzusammensetzung vorzubeugen, kann diese einen Polymerisationsinhibitor aufweisen. Als Polymerisationsinhibitoren kommen anionische und radikalische Polymerisationsinhibitoren gleichermaßen in Betracht. Im Falle eines anionischen Polymerisationsinhibitors handelt es sich in der Regel um ein saures Gas, eine protonische Säure oder ein Anhydrid davon. Beispiele für geeignete anionische Polymerisationsinhibitoren sind aus der Gruppe bestehend aus Schwefeldioxid, Phosphorpentoxid, Bortrifluorid, Distickstoffmonooxid, Chlorwasserstoff, Chlorwasserstoffsäure, Schwefelsäure, Phosphorsäure, organische Sulfonsäuren und/oder Anhydride davon, organische Carbonsäuren und/oder Anhydride davon, Säurechloride und Kombinationen davon ausgewählt. Erfindungsgemäß können anionische Polymerisationsinhibitoren in der Klebstoffzusammensetzung in einem Anteil zwischen 50 und 3.000 ppm enthalten sein.

Beispiele für geeignete radikalische Polymerisationsinhibitoren sind Hydrochinon, t-butyl-Hydroxyanisol (BHA), 2,6-di-tert-butyl-4-methylphenol (BHT), 3,5-di-tert-butyl-4-hydroxytoluol, t-Butylcatechinon, Paramethoxyphenol oder Kombinationen davon. Radikalische Polymerisationsinhibitoren können in der Klebstoffzusammensetzung einen Anteil zwischen 500 und 5.000 ppm aufweisen.

In einer weiteren Ausführungsform kann die Klebstoffzusammensetzung Formaldehydfänger aufweisen. Diese binden beim Abbau von Polycyanoacrylat entstehendes Formaldehyd und können beispielsweise aus der Gruppe bestehend aus Sulfite, Bisulfite, Ammoniumsulfite, Harnstoffsalze und Kombinationen davon ausgewählt sein.

Zur Ausbildung eines möglichst gleichmäßigen Klebstoffüberzuges auf dem Saugkörper kann es weiterhin vorgesehen sein, dass die Klebstoffzusammensetzung eine Carbonsäure, insbesondere Fettsäure, aufweist. Bevorzugt besitzt die Klebstoffzusammensetzung einen Carbonsäureanteil ≥ 0,1 Gew.-%, insbesondere zwischen 0,1 und 2 Gew.-%, vorzugsweise 0,1 und 1 Gew.-%, bezogen auf das Gesamtgewicht der Klebstoffzusammensetzung. Bevorzugte Carbonsäuren sind aus der Gruppe bestehend aus Myristinsäure, Palmitinsäure, Stearinsäure, Ölsäure, Linolsäure, Linolensäure, Salze davon und Kombinationen davon ausgewählt.

Bei dem erfindungsgemäß vorgesehenen Polymerisationsinitiator handelt es sich um einen nukleophilen Polymerisationsinitiator. Der Polymerisationsinitiator kann eine anorganische Verbindung bzw. ein anorganisches Salz, eine organische Verbindung bzw. ein organisches Salz oder eine Kombination davon sein. Bevorzugt ist der Polymerisationsinitiator eine stickstoffhaltige Verbindung.

Geeignete anorganische Polymerisationsinitiatoren können aus der Gruppe bestehend aus Aluminate, Borate, Carbonate, beispielsweise Natriumcarbonat und/oder Calciumcarbonat, Bicarbonate, beispielsweise Natriumbicarbonat und/oder Calciumcarbonat, Hydroxide, Halogenide, Oxide, Sulfonate, Sulfate und Kombinationen davon ausgewählt sein.

Der Polymerisationsinitiator ist in einer besonders bevorzugten Ausführungsform eine organische Verbindung. Bevorzugt weist der Polymerisationsinitiator primäre Amingruppen (sogenannte Aminogruppen), sekundäre Amingruppen, tertiäre Amingruppen, Imingruppen, Guanidingruppen oder Kombinationen davon auf.

In einer bevorzugten Ausführungsform ist der Polymerisationsinitiator ein Amin. Das Amin kann ein niedermolekulares oder hochmolekulares, insbesondere polymeres, Amin sein. Des Weiteren kann es sich bei dem Polymerisationsinitiator um ein in Bezug auf Amingruppen mono-, bi-, oligo- oder multifunktionelles Amin handeln. Mit anderen Worten kann es erfindungsgemäß vorgesehen sein, dass der Polymerisationsinitiator ein Di-, Oligo- oder Polyamin ist. Geeignete niedermolekulare Diamine können beispielsweise aus der Gruppe bestehend aus Ethylendiamin, 1,2-Diaminopropan, 1,3-Diaminopropan und Kombinationen davon ausgewählt sein.

In einer weitergehenden Ausführungsform ist der Polymerisationsinitiator ein biogenes Amin. Unter einem biogenen Amin soll im Sinne der vorliegenden Erfindung ein Amin verstanden werden, welches durch einen Decarboxylierungsvorgang aus einer Aminosäure, vorzugsweise einer proteinogenen Aminosäure, entstanden ist. Beispiele für geeignete biogene Amine sind Histamin, Tryptamin, Agmantin, Putrescin, Cadaverin, Spermin, Spermidin, Salze davon und/oder Kombinationen davon.

In einer weiteren Ausführungsform ist der Polymerisationsinitiator ein heterozyklisches Amin, welches vorzugsweise ausgewählt ist aus der Gruppe bestehend aus Pyridin, Pyrazin, Pyrimidin, Purin, Nukleobasen, Nukleoside, Nukleotide, Salze davon, Derivate davon und Kombinationen davon. Bevorzugte Ausführungsformen umfassen 2-Methoxypyridin, 2,2-Dipyridyldisulfit, 3,5-Dichlorpyridin, 3,5-Dibrompyridin, 4-Cyanopyridin, 2,3-Dimethoxypyrazin, 2,3-Dimethylpyrazin, 2,5-Dimethylpyrazin, Trimethylpyrazin, 2-Methoxy-3-methylpyrazin, Coffein, Harnstoff, Adenin, Guanin, Cytosin, 5-Methylcytosin, Thymin, Uracil, Hypoxanthin, Xanthin, Adenosin, Desoxyadenosin, Guanosin, Desoxyguanosin, Cytidin, 5-Methylcytidin, Desoxycytidin, Desoxy-5-methylcytidin, Thymidin, Desoxythymidin, Uridin, Desoxyuridin, Salze davon, Derivate davon und Kombinationen davon.

In einer weiteren, besonders bevorzugten Ausführungsform handelt es sich bei dem Polymerisationsinitiator um eine Aminosäure, vorzugsweise um eine α-Aminosäure. Mischungen von Aminosäuren können ebenfalls bevorzugt sein. Vorzugsweise ist der Polymerisationsinitiator eine proteinogene bzw. kanonische Aminosäure, insbesondere eine basische Aminosäure. Unter einer basischen Aminosäure soll im Sinne der vorliegenden Erfindung eine Aminosäure verstanden werden, die eine Seitenkette mit einer basischen Gruppe aufweist. Beispiele für erfindungsgemäß bevorzugte basische Aminosäuren stellen Histidin, Arginin und/oder Lysin dar. Erfindungsgemäß kann der Polymerisationsinitiator grundsätzlich auch eine synthetische Aminosäure sein. Bevorzugt ist der Polymerisationsinitiator eine Aminosäure, welche ausgewählt ist aus der Gruppe bestehend aus Arginin, Cystein, Histidin, Lysin, Methionin, Phenylalanin, Prolin, Serin, Tryptophan, Tyrosin, Salze davon, Derivate davon und Kombinationen davon.

In einer weiteren Ausführungsform liegt der Polymerisationsinitiator als Peptid, insbesondere als Di-, Oligo- oder Polypeptid, vor. Dabei kann der Polymerisationsinitiator ein natürliches Peptid, beispielsweise Glutathion, oder ein synthetisches Peptid sein.

In einer weiteren Ausführungsform ist der Polymerisationsinitiator ein Protein, ein Derivat davon oder ein Salz davon, insbesondere ein extrazelluläres Protein oder ein Salz davon. Bevorzugte Proteine sind aus der Gruppe bestehend aus Gelatine, Gelatine-polysuccinat, Gelafundin, Collagen, Elastin, Retikulin, Fibronektin, Polylysin, insbesondere Poly-ε-lysin, Albumin, Derivate davon, Salze davon und Kombinationen davon ausgewählt.

In einer weiteren Ausführungsform ist der Polymerisationsinitiator ein Polysaccharid, insbesondere ein stickstoffhaltiges, vorzugsweise Aminogruppen tragendes, Polysaccharid. Geeignete Polysaccharide sind beispielsweise Chitosan und/oder zumindest teilweise deacetylierte Mucopolysaccharide.

In einer weiteren Ausführungsform ist der Polymerisationsinitiator ein synthetisches Polymer, welches vorzugsweise stickstoffhaltige Gruppen, insbesondere Aminogruppen, aufweist. Geeignete Polymere können beispielsweise aus der Gruppe bestehend aus Polyethylenimine, Polyhexamethylenbiguanide, Aminogruppen tragende bzw. aminierte Polyvinylalkohole, Aminogruppen tragende bzw. aminierte Polyhydroxyalkanoate, Aminogruppen tragende bzw. aminierte Polysaccharide, Aminogruppen tragende bzw. aminierte Polyethylenglykole, Salze davon, Copolymere davon und Kombinationen davon ausgewählt sein.

In einer weiteren Ausführungsform handelt es sich bei dem Polymerisationsinitiator um eine antimikrobiell wirksame Verbindung. Bei der antimikrobiell wirksamen Verbindung kann es sich um eine der bereits zuvor genannten Ausführungsformen für den Polymerisationsinitiator handeln. Bevorzugt ist ein antimikrobiell wirksamer Polymerisationsinitiator jedoch aus der Gruppe bestehend aus Polyhexamethylenbiguanid, Akazid^{®}, Cyclohexidin, Cetyltrimethylammoniumbromid, Octinidin, Benzalko-niumchlorid, Cetylpyridiniumchlorid, Polylysin, insbesondere Poly-ε-lysin, Uracil, Spermin, Tryptamin, Polyethylenimin, Salze davon und Kombinationen davon ausgewählt.

In der Regel liegen in dem erfindungsgemäßen Kit die polymerisierbare Klebstoffzusammensetzung und der Polymerisationsinitiator räumlich bzw. physikalisch getrennt voneinander vor. Auf diese Weise kann einer vorzeitigen Polymerisation bzw. Aushärtung der Klebstoffzusammensetzung vorgebeugt werden. Beispielsweise können die polymerisierbare Klebstoffzusammensetzung und der Polymerisationsinitiator in unterschiedlichen Kompartimenten bzw. Behältnissen einer Austragsvorrichtung, wie sie üblicherweise zum Austrag von medizinischen Klebstoffzusammensetzungen verwendet wird, enthalten sein.

In einer weiteren Ausführungsform ist der Saugkörper mit dem Polymerisationsinitiator ausgerüstet. Dabei kann der Polymerisationsinitiator auf einer äußeren und/oder inneren Oberfläche des Saugkörpers verteilt sein. Beispielsweise kann der Polymerisationsinitiator in Form einer Beschichtung oder Imprägnierung, gegebenenfalls auch nur in Form einer teilweisen Beschichtung bzw. Imprägnierung, auf einer äußeren und/oder inneren Oberfläche des Saugkörpers vorliegen. Insbesondere können Poren des Saugkörpers vollständig mit einer Schicht des Polymerisationsinitiators überzogen bzw. bedeckt sein und/oder Porenstege des Saugkörpers zumindest teilweise, insbesondere vollständig, von einer Schicht des Polymerisationsinitiators ummantelt sein.

Der Polymerisationsinitiator kann in flüssiger Form, insbesondere als flüssige Dispersion, Suspension oder Lösung, vorliegen. Bevorzugt ist es, wenn der Polymerisationsinitiator in Form einer wässrigen Dispersion, wässrigen Lösung oder wässrigen Suspension vorliegt. Besonders bevorzugt ist eine wässrige Lösung des Polymerisationsinitiators.

In einer alternativen Ausführungsform liegt der Polymerisationsinitiator als Gel, insbesondere Hydrogel, Paste oder als Feststoff, insbesondere in partikulärer Form, vorzugsweise als Puder, Pulver oder Granulat, vor. Erfindungsgemäß ist es besonders bevorzugt, wenn der Polymerisationsinitiator als partikulärer Polymerisationsinitiator vorliegt. Zum Beispiel kann der Polymerisationsinitiator in Form von sprühfähigen Partikeln vorliegen. Der Polymerisationsinitiator kann eine Partikelgröße zwischen 0,01 und 1000 µm, insbesondere 0,1 und 1000 µm, bevorzugt 1 und 1000 µm, aufweisen. Die Partikel können sphärisch, sphäroid oder unregelmäßig geformt sein. Des Weiteren kann der Polymerisationsinitiator lyophilisiert, beispielsweise als lyophilisiertes Pulver oder lyophilisiertes Granulat, vorliegen.

In einer weiteren alternativen Ausführungsform liegt der Polymerisationsinitiator als Flächengebilde, vorzugsweise ebenes Flächengebilde, insbesondere als Folie, Membran, Kompresse oder dergleichen, vor.

In einer weiteren Ausführungsform umfasst das erfindungsgemäße Kit auch eine Austragsvorrichtung für die polymerisierbare Klebstoffzusammensetzung und den Polymerisationsinitiator. Bevorzugt sind die polymerisierbare Klebstoffzusammensetzung und der Polymerisationsinitiator in unterschiedlichen Kompartimenten bzw. Behältnissen der Austragsvorrichtung enthalten. Bei der Austragsvorrichtung kann es sich beispielsweise um eine Zweikammer- bzw. Zwillingsspritze handeln, wobei die polymerisierbare Klebstoffzusammensetzung in der einen Kammer und der Polymerisationsinitiator in der anderen Kammer der Spritze enthalten ist. Bevorzugt umfasst die Austragsvorrichtung eine Mischungseinheit für die polymerisierbare Klebstoffzusammensetzung und den Polymerisationsinitiator. Bei der Mischungseinheit kann es sich um eine statische Mischungseinheit, eine dynamische Mischungseinheit oder um einen Sprühkopf handeln. Die Mischungseinheit ist ferner vorzugsweise austauschbar. Erfindungsgemäß ist es besonders vorteilhaft, wenn die Mischungseinheit auf die Austragsvorrichtung aufsteckbar ist. Des Weiteren kann die Mischungseinheit zur Vermischung von ausschließlich flüssigen Komponenten (flüssig/flüssig-System) und/oder von festen und flüssigen Komponenten (fest/flüssig-System) ausgebildet sein.

Bevorzugt ist die Austragsvorrichtung als Sprühvorrichtung ausgebildet. Ein Versprühen der polymerisierbaren Klebstoffzusammensetzung und des Polymerisationsinitiators hat den Vorteil, dass sich hierdurch die bei Polymerisation bzw. Aushärtung der Klebstoffzusammensetzung auftretende Exothermie verringern lässt. Das Sprühen kann durch Druckluft, ein geeignetes Gas, beispielsweise Stickstoff oder Kohlendioxid, ein Pumpsprühsystem oder durch Druckbeaufschlagung eines Spritzenstempels bzw. -kolbens erfolgen. Um einer unerwünschten, vorzeitigen Polymerisation der Klebstoffzusammensetzung in der Austragsvorrichtung vorzubeugen, kann es im Falle einer Sprühvorrichtung erwünscht sein, wenn eine Vermischung der polymerisierbaren Klebstoffzusammensetzung und des Polymerisationsinitiators erst außerhalb der Austragsvorrichtung stattfindet. In der Regel geschieht dies dadurch, dass sich Sprühkegel der polymerisierbaren Klebstoffzusammensetzung und des Polymerisationsinitiators erst außerhalb der Austragsvorrichtung überlappen. Erfindungsgemäß kann es jedoch ebenso bevorzugt sein, dass eine Mischung der Klebstoffzusammensetzung und des Polymerisationsinitiators in einer Spraydüse erfolgt. Dadurch ist zum Einen eine bessere Durchmischung von Klebstoffzusammensetzung und Polymerisationsinitiator möglich. Zum Anderen lässt sich ein fokussierter Strahl erzeugen, was abhängig von der zu behandelnden Wunde von Vorteil sein kann. Weiterhin kann die Austragsvorrichtung als Dosiervorrichtung ausgebildet sein, welche beispielsweise Arretiereinheiten als Äquivalente für Dosiseinheiten aufweist.

In einer weiteren Ausführungsform weist das Kit, insbesondere der Saugkörper und/oder die polymerisierbare Klebstoffzusammensetzung, Wirkstoffe, insbesondere biologische und/oder medizinische bzw. pharmazeutische Wirkstoffe, gegebenenfalls auch in Form von Arzneimitteln und/oder pharmazeutischen Zusammensetzungen, auf. Bevorzugte Wirkstoffe sind aus der Gruppe bestehend aus antimikrobielle, insbesondere antibiotische, Wirkstoffe, antiseptische Wirkstoffe, desinfizierende Wirkstoffe, entzündungshemmende Wirkstoffe, wundheilungsfördernde Wirkstoffe, antimietische Wirkstoffe, geruchsbekämpfende Wirkstoffe, schmerzlindernde Wirkstoffe, dermatologische Wirkstoffe, zellwachstumsfördernde Wirkstoffe, zellrekrutierende Wirkstoffe, zelldifferenzierende Wirkstoffe, zelladhäsive Wirkstoffe, Salze, insbesondere Fettsalze, davon und Kombinationen davon ausgewählt. In Bezug auf mögliche wundheilungsfördernde Wirkstoffe sei beispielhaft auf Hyaluronsäure und/oder Zinkverbindungen, insbesondere Zinksalze, beispielsweise Zinkoxid und/ oder Zinkhyaluronat, verwiesen. Besonders bevorzugt sind antimikrobielle Wirkstoffe. Als antimikrobielle Wirkstoffe kommen neben den bereits in dieser Beschreibung erwähnten antimikrobiellen Verbindungen auch antimikrobiell wirksame Metalle, Metalllegierungen und/oder Metallsalze in Betracht. Bevorzugt sind die antimikrobiellen Wirkstoffe aus der Gruppe bestehend aus Kupfer, Zink, Titan, Silber, Gold, Platin, Legierungen davon, Salze davon und Kombinationen davon ausgewählt.

Im Folgenden werden verschiedene Möglichkeiten der Vakuumversiegelungstherapie unter Verwendung des erfindungsgemäßen Kits erläutert.

Eine Möglichkeit sieht beispielsweise vor, dass der Saugkörper zunächst auf eine Wunde, welche mittels der Vakuumversiegelung behandelt werden soll, aufgelegt und anschließend mit der polymerisierbaren Klebstoffzusammensetzung und dem Polymerisationsinitiator in Kontakt gebracht wird, vorzugsweise mittels Aufsprühen der Klebstoffzusammensetzung und des Polymerisationsinitiators auf den Saugkörper.

Eine alternative Möglichkeit sieht beispielsweise vor, dass der Saugkörper vor dem Auflegen auf eine mittels Vakuum zu versiegelnde Wunde in eine flüssige Dispersion, Lösung oder Suspension des Polymerisationsinitiators, vorzugsweise in eine wässrige Lösung des Polymerisationsinitiators, eingetaucht wird. Anschließend wird der Saugkörper auf die Wundfläche aufgelegt und mit der polymerisierbaren Klebstoffzusammensetzung in Kontakt gebracht, vorzugsweise mittels Aufsprühen.

Eine weitere alternative Möglichkeit sieht beispielsweise vor, dass ein bereits mit einem Polymerisationsinitiator ausgerüsteter Saugkörper auf eine zu behandelnde Wunde aufgelegt und anschließend die polymerisierbare Klebstoffzusammensetzung hinzugegeben, vorzugsweise aufgesprüht, wird.

Noch eine weitere Möglichkeit sieht beispielsweise vor, den Saugkörper nach Auflegen auf eine Wunde mit einer Folie zu bedecken und anschließend die Folie mit der polymerisierbaren Klebstoffzusammensetzung und dem Polymerisationsinitiator in Kontakt zu bringen, vorzugsweise mittels Aufsprühen der Klebstoffzusammensetzung und des Polymerisationsinitiators auf die Folie.

Bevorzugt besitzt die polymerisierbare Klebstoffzusammensetzung auf dem Saugkörper in Gegenwart des Polymerisationsinitiators eine Topf- bzw. Gelzeit zwischen 1 und 300 s, insbesondere 1 und 120 s, vorzugsweise 1 und 60 s. In der Regel ist der Saugkörper nach einer solchen Topf- bzw. Gelzeit ausreichend an den umliegenden Wundrändern fixiert und insbesondere vakuumdicht verschlossen. Über einen Drainageschlauch ist dann eine Ableitung bzw. Abführung von Körperflüssigkeiten, welche von dem Saugkörper aufgenommen werden, möglich. Zu diesem Zweck wird der Drainageschlauch, wie bereits vorstehend beschrieben, üblicherweise an eine geeignete Vakuumquelle verbunden.

Grundsätzlich kann das erfindungsgemäße Kit zur Versorgung bzw. Behandlung von allen denkbaren topologischen, d.h. oberflächlichen, Wunden und/oder inneren Wunden eingesetzt werden. Das Kit eignet sich insbesondere für Wunden mit schwierigen Oberflächengeometrien, beispielsweise für Wundoberflächen, auf welche keine Folien aufgelegt werden können. Besonders geeignet ist das Kit zur Versorgung bzw. Behandlung von sekundär heilenden Wunden, mäßig bis stark exsudierenden Wunden, Dekubitus, Ulcera (offene Wunden), post-operativen Wunden, Spalthautentnahmestellen, diabetisches Fusssyndrom und/oder oberflächliche bis tiefe Hautwunden.

Weitere Merkmale und Vorteile der Erfindung ergeben sich aus den nachfolgend beschriebenen Ausführungsformen in Form einer Figurenbeschreibung, einer dazugehörigen Figur sowie in Form von Beispielen in Verbindung mit den Unteransprüchen. Hierbei können einzelne Merkmale der Erfindung alleine oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

In der einzigen Figur ist schematisch gezeigt:

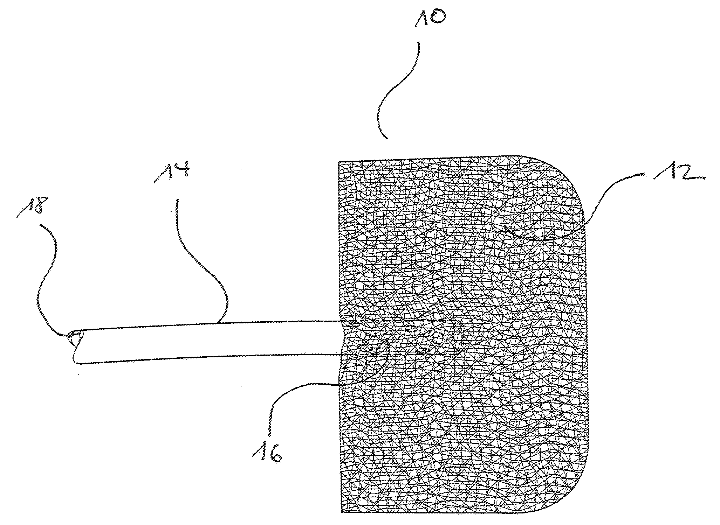

- Figur 1:: ein Saugkörper zum Aufsaugen von Körperflüssigkeiten wie er in einem erfindungsgemäßen Kit zum Einsatz kommt.

### Figurenbeschreibunq

Die Figur 1 zeigt schematisch einen Saugkörper 10 wie er für einen Einsatz im Rahmen eines erfindungsgemäßen Kits vorgesehen ist. Der Saugkörper 10 besitzt eine offenporöse Struktur 12, bei welcher es sich bevorzugt um eine Schwamm- oder Schaumstruktur handelt. Bevorzugt ist der Saugkörper 10 bzw. dessen offenporöse Struktur 12 aus Polyurethan gebildet.

Durch die offenporöse Struktur 12 ist der Saugkörper 10 in der Lage, Körperflüssigkeiten, beispielsweise Wundflüssigkeiten aus einem Wundmilieu, aufzunehmen.

Des Weiteren weist der Saugkörper 10 auch einen Drainageschlauch 14 auf. Beide Enden des Drainageschlauches 14 sind in der Regel offen. Ein Teil des Drainageschlauches 14 ragt dabei in die offenporöse Struktur 12 hinein. Dieser Teil des Drainageschlauches 14 weist vorzugsweise Perforationen bzw. Löcher 16 in der Schlauchwand auf, um einen besseren Flüssigkeitsaustausch zwischen der offenporösen Struktur 12 und dem Drainageschlauch 14 zu ermöglichen.

An seinem freien Ende 18 kann der Drainageschlauch 14 an eine Saug- oder Vakuumpumpe angeschlossen werden (nicht dargestellt). Um zum Beispiel eine topologische Wunde mittels Vakuum zu versiegeln und zu drainieren, ist es bevorzugt, dass der Saugkörper 10 mit seiner offenporösen Struktur 12 auf die Wunde aufgelegt wird und anschließend mit einer polymerisierbaren Klebstoffzusammensetzung, welche Cyanoacrylat-Monomere als polymerisierbare Komponenten enthält, sowie mit einem Polymerisationsinitiator, beispielsweise Histidin, großflächig besprüht wird.

Bezüglich alternativer Formen der Vakuumversiegelung und -drainage von Wunden mittels des erfindungsgemäßen Kits wird auf die entsprechenden Ausführungen in der vorangegangenen Beschreibung Bezug genommen.

Nach Polymerisation bzw. Aushärtung der Klebstoffzusammensetzung ist die offenporöse Struktur 12 mit besonderem Vorteil vollständig von einem polymeren Film aus Polycyanoacrylat überzogen. Auf diese Weise kann zum Einen ein luftdichter Verschluss der unter der offenporösen Struktur 12 liegenden Wunde, zum Anderen eine Fixierung der offenporösen Struktur 12 bzw. des Saugkörpers 10 an den Wundrändern erzielt werden.

Über eine an das freie Ende 18 des Drainageschlauches 14 angeschlossene Saug- oder Vakuumpumpe kann nun ein Unterdruck angelegt werden, um die vom Saugkörper 10 aufgesogenen Körperflüssigkeiten über den Drainageschlauch 14 aus einem Wundmilieu abzuleiten.

### Beispiel

Ein Polyurethanschaum (Askina^{®}) mit den Ausmaßen 5 x 7 cm wurde halbiert. Anschließend wurde in jede Schaumhälfte ein Schlauchstück gesteckt, wobei das Schlauchende, welches in die Schaumhälften gesteckt wurde, über einen Bereich von 0,5 cm perforiert war.

Die so präparierten Polyurethanschaumhälften wurden auf Fleischstücke mit einer Größe von ca. 10 x 10 cm² gelegt und mit einem auf n-Butylcyanoacrylat (Histacryl^{®}) und Glycerintriacetat (Weichmacher) basierenden Zweikomponentenkleber und einer wässrigen Histidinlösung als Initiatorlösung komplett bedeckt. Das Verhältnis von n-Butylcyanoacrylat zu Glycerintriacetat im Zweikomponentenkleber betrug 60 Gew.-% zu 40 Gew.-%. Die Lösung wies eine Konzentration an Histidin von 1 Gew.-%, bezogen auf das Gesamtgewicht der Lösung, auf.

Innerhalb weniger Sekunden waren die Fleischstücke im Wesentlichen vollständig von dem ausgehärteten Zweikomponentenkleber (Polycyanoacrylat) überzogen.

Anschließend wurde das freie Ende des Schlauchstückes an eine Vakuumvorrichtung angeschlossen. Das Anlegen eines Vakuums bzw. Unterdrucks führte zunächst zur Komprimierung des Polyurethanschaums sowie des darüberliegenden polymerisierten Überzuges. Anschließend wurde den Fleischstücken in Folge der Saugwirkung überschüssige Feuchtigkeit entzogen. Hierbei konnte ein Vakuum bis zu einem Restdruck von 5 mbar erzeugt werden.

Die Durchführung des vorhergehend beschriebenen Versuchs mit Tributylacetylcitrat anstelle von Glycerintriacetat als Weichmacher führte ebenfalls zu sehr guten Ergebnissen. So konnte nach Aushärtung von Histoacryl^{®} auch in diesem Fall ein zufriedenstellendes Vakuum erzeugt werden.

In einem Vergleichsversuch wurden Polyurethanschaumhälften, wie sie oben beschrieben sind, auf Fleischstücke aufgelegt und lediglich mit einem Zweikomponentenkleber der oben beschriebenen Art, d.h. ohne Zusatz einer wässrigen Histidinlösung, bedeckt. Hierdurch ließ sich kein zufriedenstellendes Vakuum bzw. kein zufriedenstellender Unterdruck erzeugen. Auch durch Verwendung einer doppelten Menge des Zweikomponentenklebers konnte kein ausreichendes Vakuum bzw. kein ausreichender Unterdruck erzeugt werden.

## Patentansprüche

1. Medizinisches Kit, insbesondere zur Vakuumversiegelung und -drainage von Wunden, umfassend die folgenden Komponenten:
1.1 einen Saugkörper zum Aufsaugen von Körperflüssigkeiten, insbesondere Blut, Wundflüssigkeiten oder dergleichen,
1.2 eine polymerisierbare Klebstoffzusammensetzung und
1.3 einen Polymerisationsinitiator, wobei die Klebstoffzusammensetzung Cyanoacrylat-Monomere gemäß Formel (I) 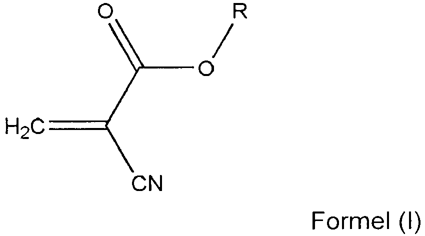
aufweist, wobei R eine substituierte oder unsubstituierte, geradkettige, verzweigte oder cyclische Alkyl-, Haloalkyl-, Alkoxyalkyl-, Alkenyl-, Allyl- oder Alkinylgruppe ist, die vorzugsweise 1 bis 18 Kohlenstoffatome umfasst, und/oder eine aromatische Gruppe und/oder eine Acylgruppe aufweist, und es sich bei dem Polymerisationsinitiator um einen nukleophilen Polymerisationsinitiator handelt.

2. Medizinisches Kit nach Anspruch 1, **dadurch gekennzeichnet, dass** der Saugkörper eine offenporöse Struktur, vorzugsweise Poren mit einem Durchmesser zwischen 50 und 1.500 µm, insbesondere 200 und 1.000 µm, vorzugsweise 250 und 650 µm, aufweist.

3. Medizinisches Kit nach Anspruch 1 oder 2, **dadurch gekennzeichnet, dass** der Saugkörper als Folie, Schwamm oder Schaum ausgebildet ist.

4. Medizinisches Kit nach einem der vorhergehenden Ansprüche, **dadurch gekennzeichnet, dass** der Saugkörper aus einem bioverträglichen Polymer gebildet ist, insbesondere ausgewählt aus der Gruppe bestehend aus Polyurethane, Polyester, Polyvinylalkohole, Polyhydroxyalkanoate, Polysaccharide, Salze davon, Copolymere davon und Mischungen davon.

5. Medizinisches Kit nach einem der vorhergehenden Ansprüche, **dadurch gekennzeichnet, dass** der Saugkörper einstückig mit einem Drainageschlauch verbunden ist, wobei vorzugsweise ein perforiertes Ende des Drainageschlauches von dem Saugkörper umgeben bzw. ummantelt ist.

6. Medizinisches Kit nach einem der vorhergehenden Ansprüche, **dadurch gekennzeichnet, dass** die Klebstoffzusammensetzung ferner einen Weichmacher, insbesondere einen Weichmacheranteil zwischen 5 und 75 Gew.-%, insbesondere 10 und 70 Gew.-%, vorzugsweise 15 und 50 Gew.-%, aufweist, bezogen auf das Gesamtgewicht der Klebstoffzusammensetzung.

7. Medizinisches Kit nach einem der vorhergehenden Ansprüche, **dadurch gekennzeichnet, dass** es sich bei dem Polymerisationsinitiator um einen stickstoffhaltigen Polymerisationsinitiator handelt.

8. Medizinisches Kit nach einem der vorhergehenden Ansprüche, **dadurch gekennzeichnet, dass** der Polymerisationsinitiator eine organische Verbindung ist, vorzugsweise ausgewählt ist aus der Gruppe bestehend aus Amine, Aminosäuren, Peptide, Oligopeptide, Polypeptide, Proteine, Polysaccharide, stickstoffhaltige synthetische Polymere, Salze davon und Kombinationen davon.

9. Medizinisches Kit nach einem der vorhergehenden Ansprüche, **dadurch gekennzeichnet, dass** der Polymerisationsinitiator eine proteinogene Aminosäure und/oder ein biogenes Amin ist, vorzugsweise ausgewählt aus der Gruppe bestehend aus Arginin, Cystein, Histidin, Lysin, Methionin, Phenylalanin, Prolin, Serin, Tryptophan, Tyrosin, Histamin, Tryptamin, Agmatin Putrescin, Cadaverin, Spermin, Spermidin, Salze davon und Kombinationen davon.

10. Medizinisches Kit nach einem der vorhergehenden Ansprüche, **dadurch gekennzeichnet, dass** der Polymerisationsinitiator eine antimikrobiell wirksame Verbindung ist, vorzugsweise ausgewählt aus der Gruppe bestehend aus Polyhexamethylenbiguanid, Akazid^{®}, Cyclohexidin, Cetyltrimethylammoniumbromid, Octinidin, Benzalko-niumchlorid, Cetylpyridiniumchlorid, Polylysin, insbesondere Poly-ε-lysin, Uracil, Spermin, Tryptamin, Polyethylenimin, Salze davon und Kombinationen davon

11. Medizinisches Kit nach einem der vorhergehenden Ansprüche, **dadurch gekennzeichnet, dass** die polymerisierbare Klebstoffzusammensetzung und der Polymerisationsinitiator räumlich getrennt voneinander vorliegen.

12. Medizinisches Kit nach einem der vorhergehenden Ansprüche, **dadurch gekennzeichnet, dass** der Polymerisationsinitiator in flüssiger Form, vorzugsweise als wässrige Lösung, vorliegt.

13. Medizinisches Kit nach einem der Ansprüche 1 bis11, **dadurch gekennzeichnet, dass** der Polymerisationsinitiator als Gel, insbesondere Hydrogel, Paste oder als Feststoff, insbesondere als lyophilisierter Feststoff, vorzugsweise als Puder, Pulver oder Granulat, vorliegt.

14. Medizinisches Kit nach einem der vorhergehenden Ansprüche, **dadurch gekennzeichnet, dass** das Kit ferner eine Austragsvorrichtung zum Austrag von medizinischen Klebstoffzusammensetzungen, vorzugsweise eine Doppelkammer-Austragsvorrichtung, aufweist.

## Claims

1. Medical kit, in particular for negative-pressure wound therapy and wound drainage, comprising the following components:
1.1 an absorbent matter for absorbing body fluids, in particular blood, wound fluids or the like,
1.2 a polymerizable adhesive composition, and
1.3 a polymerization initiator, wherein the adhesive composition includes cyanoacrylate monomers according to formula (I) 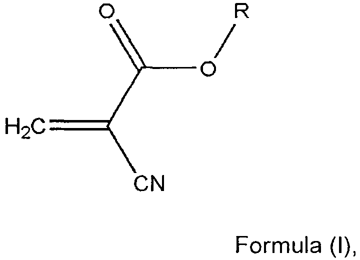 wherein R is a substituted or unsubstituted, straight-chain, branched or cyclic alkyl, haloalkyl, alkoxyalkyl, alkenyl, allyl or alkynyl group, which preferably comprises 1 to 18 carbon atoms, and/or includes an aromatic group and/or an acyl group, and the polymerization initiator is a nucleophilic polymerization initiator.

2. Medical kit according to claim 1, **characterized in that** the absorbent matter has an open-porous structure, preferably having pores with a diameter between 50 and 1,500 µm, in particular 200 and 1,000 µm, preferably 250 and 650 µm.

3. Medical kit according to claim 1 or 2, **characterized in that** the absorbent matter is in the form of a film, sponge or foam.

4. Medical kit according to any of the preceding claims, **characterized in that** the absorbent matter is composed of a biocompatible polymer, in particular selected from the group consisting of polyurethanes, polyesters, polyvinyl alcohols, polyhydroxyalkanoates, polysaccharides, salts thereof, copolymers thereof, and mixtures thereof.

5. Medical kit according to any of the preceding claims, **characterized in that** the absorbent matter is integrally connected to a drainage tube, wherein preferably a perforated end of the drainage tube is covered or sheathed by the absorbent matter.

6. Medical kit according to any of the preceding claims, **characterized in that** the adhesive composition further includes a plasticizer, in particular a proportion of plasticizer between 5 and 75 % by weight, in particular 10 and 70 % by weight, preferably 15 and 50 % by weight, in relation to the total weight of the adhesive composition.

7. Medical kit according to any of the preceding claims, **characterized in that** the polymerization initiator is a nitrogenous polymerization initiator.

8. Medical kit according to any of the preceding claims, **characterized in that** the polymerization initiator is an organic compound, preferably selected from the group consisting of amines, amino acids, peptides, oligopeptides, polypeptides, proteins, polysaccharides, nitrogenous synthetic polymers, salts thereof, and combinations thereof.

9. Medical kit according to any of the preceding claims, **characterized in that** the polymerization initiator is a proteinogenic amino acid and/or a biogenic amine, preferably selected from the group consisting of arginine, cysteine, histidine, lysine, methionine, phenylalanine, proline, serine, tryptophan, tyrosine, histamine, tryptamine, agmatine, putrescine, cadaverine, spermine, spermidine, salts thereof, and combinations thereof.

10. Medical kit according to any of the preceding claims, **characterized in that** the polymerization initiator is an antimicrobially active compound, preferably selected from the group consisting of polyhexamethylene biguanide, Akazid®, cyclohexidine, cetyltrimethylammonium bromide, octinidine, benzalkonium chloride, cetylpyridinium chloride, polylysine, in particular poly-ε-lysine, uracil, spermine, tryptamine, polyethylenimine, salts thereof, and combinations thereof.

11. Medical kit according to any of the preceding claims, **characterized in that** the polymerizable adhesive composition and the polymerization initiator are present in separate units.

12. Medical kit according to any of the preceding claims, **characterized in that** the polymerization initiator is present in liquid form, preferably in an aqueous solution.

13. Medical kit according to any of claims 1 to 11, **characterized in that** the polymerization initiator is a gel, in particular hydrogel, paste or solid, in particular lyophilized solid, preferably particles, powder or granules.

14. Medical kit according to any of the preceding claims, **characterized in that** the kit further includes a dispenser device for dispensing medical adhesive compositions, preferably a dual-chamber dispenser device.

## Revendications

1. Kit médical, en particulier pour le scellement et le drainage sous vide de plaies, comprenant les composants suivants :
1.1 un corps d'aspiration, pour aspirer des fluides corporels, en particulier le sang, les exsudats ou analogues,
1.2 une composition adhésive polymérisable, et
1.3 un amorceur de polymérisation, la composition adhésive comprenant des cyanoacrylates monomères de formule (I) 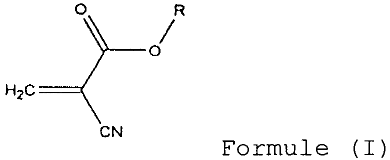 dans laquelle R est un groupe alkyle, halogènalkyle, alcoxyalkyle, alcényle, allyle ou alcynyle, substitué ou non substitué, à chaîne droite, ramifiée ou cyclique, qui de préférence comprend 1 à 18 atomes de carbone, et/ou présente un groupe aromatique et/ou un groupe acyle, et, pour ce qui concerne l'amorceur de polymérisation, il s'agit d'un amorceur de polymérisation nucléophile.

2. Kit médical selon la revendication 1, **caractérisé en ce que** le corps d'aspiration présente une structure à pores ouverts, de préférence des pores ayant un diamètre compris entre 50 et 1500 µm, en particulier entre 200 et 1000 µm, de préférence entre 250 et 650 µm.

3. Kit médical selon la revendication 1 ou 2, **caractérisé en ce que** le corps d'aspiration est configuré sous forme d'une feuille, d'une éponge ou d'une mousse.

4. Kit médical selon l'une des revendications précédentes, **caractérisé en ce que** le corps d'aspiration est formé d'un polymère biocompatible, choisi en particulier dans le groupe consistant en les polyuréthannes, les polyesters, les poly(alcools vinyliques), les polyhydroxyalcanoates, les polysaccharides, les sels de ceux-ci, les copolymères de ceux-ci et les mélanges de ceux-ci.

5. Kit médical selon l'une des revendications précédentes, **caractérisé en ce que** le corps d'aspiration est relié d'un seul tenant à un tuyau de drainage, de préférence une extrémité perforée du tuyau de drainage étant entourée ou enveloppée par le corps d'aspiration.

6. Kit médical selon l'une des revendications précédentes, **caractérisé en ce que** la composition adhésive comprend en outre un plastifiant, en particulier une proportion de plastifiant comprise entre 5 et 75 % en poids, en particulier entre 10 et 70 % en poids, de préférence entre 15 et 50 % en poids, par rapport au poids total de la composition adhésive.

7. Kit médical selon l'une des revendications précédentes, **caractérisé en ce que**, pour ce qui concerne l'amorceur de polymérisation, il s'agit d'un amorceur de polymérisation azoté.

8. Kit médical selon l'une des revendications précédentes, **caractérisé en ce que** l'amorceur de polymérisation est un composé organique, choisi de préférence dans le groupe consistant en les amines, les acides aminés, les peptides, les oligopeptides, les polypeptides, les protéines, les polysaccharides, les polymères synthétiques azotés, les sels de ceux-ci et les combinaisons de ceux-ci.

9. Kit médical selon l'une des revendications précédentes, **caractérisé en ce que** l'amorceur de polymérisation est un acide aminé protéinogène et/ou une amine biogène, de préférence choisis dans le groupe consistant en l'arginine, la cystéine, l'histidine, la lysine, la méthionine, la phénylalanine, la proline, la sérine, le tryptophane, la tyrosine, l'histamine, la tryptamine, l'agmatine, la putrescine, la cadavérine, la spermine, la spermidine, les sels de ceux-ci et les combinaisons de ceux-ci.

10. Kit médical selon l'une des revendications précédentes, **caractérisé en ce que** l'amorceur de polymérisation est un composé à activité antimicrobienne, choisi de préférence dans le groupe consistant en le polyhexaméthylènebiguanide, l'Akazid®, la cyclohexidine, le bromure de cétyltriméthylammonium, l'octinidine, le chlorure de benzalkonium, le chlorure de cétylpyridinium, la polylysine, en particulier la poly-ε-lysine, l'uracile, la spermine, la tryptamine, la polyéthylène-imine, les sels de ceux-ci et les combinaisons de ceux-ci.

11. Kit médical selon l'une des revendications précédentes, **caractérisé en ce que** la composition adhésive polymérisable et l'amorceur de polymérisation sont présents spatialement séparés l'un de l'autre.

12. Kit médical selon l'une des revendications précédentes, **caractérisé en ce que** l'amorceur de polymérisation se présente sous forme liquide, en particulier en solution aqueuse.

13. Kit médical selon l'une des revendications 1 à 11, **caractérisé en ce que** l'amorceur de polymérisation se présente sous forme d'un gel, en particulier d'un hydrogel, d'une pâte, ou d'une matière solide, en particulier sous forme d'un solide lyophilisé, de préférence sous forme d'une poudre, d'un matériau pulvérulant ou d'un granulé.

14. Kit médical selon l'une des revendications précédentes, **caractérisé en ce que** le kit comprend en outre un dispositif distributeur, pour distribuer des compositions adhésives médicales, de préférence un dispositif distributeur à double compartiment.
